# FASCICULE DE BREVET EUROPEEN

(11) **EP 4 619 063 B1**
(45) Date de publication et mention de la délivrance du brevet: **15.07.2026**
(21) Numéro de dépôt: 23822063.6
(22) Date de dépôt: 13.11.2023
(51) Int. Cl.: A61M 11/00, A61M 15/08, B05B 11/02

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 14.11.2022 FR 2211823
(43) Date de publication de la demande: 24.09.2025
(73) Titulaire: APTAR France SAS, 27110 Le Neubourg (FR)
(72) Inventeur: BELKHEROUF, Youcef, 27000 EVREUX (FR); BIZET, Bruno, 71000 SANCÉ (FR)
(74) Mandataire: CAPRI
(86) Numéro de dépôt international: PCT/FR2023/051776
(87) Numéro de publication internationale: WO 2024/105329

(56) Documents cités:
- WO-A1-2015/104511
- GB-A- 2 262 138
- US-A- 4 962 868

## Description

La présente invention concerne un dispositif de distribution de produit fluide, et plus particulièrement un dispositif de distribution de produit fluide du type unidose destiné à distribuer une seule dose.

Les dispositifs de type unidose sont bien connus dans l'état de la technique et trouvent une partie de leurs applications dans le domaine des distributeurs pharmaceutiques à utilisation nasale, dans lesquelles une dose de produit doit être distribuée dans une narine.

Le document GB2262138 divulgue un tel dispositif unidose. Pour utiliser ce dispositif, l'utilisateur place l'orifice de distribution dans sa narine et exerce une pression axiale sur le réservoir ou l'organe d'actionnement solidaire dudit réservoir. Cette pression axiale doit être suffisamment élevée pour surmonter des moyens d'accumulation d'énergie afin de garantir la distribution de la totalité de la dose.

Lors de l'application de cette force axiale relativement importante, il est difficile de contrôler précisément la position de l'extrémité du dispositif comportant l'ouverture de distribution dans la narine, et il peut arriver que le bout de la tête du dispositif vienne buter au fond de la narine, ce qui peut être gênant et/ou douloureux pour l'utilisateur.

D'autre part, du fait que ce dispositif nécessite une force minimale prédéterminée pour pouvoir être actionné, il peut être difficile à utiliser pour des personnes âgées ou peu mobiles qui peuvent avoir des difficultés à exercer cette force avec le dispositif placé dans la narine.

Par ailleurs, il existe de dispositifs de distribution de produit fluide du type unidose, éventuellement rechargeables, qui utilisent une seringue en tant que réservoir contenant la dose de produit fluide. Le document WO2015104511 décrit un dispositif de ce type.

Un inconvénient de ce type de dispositif, notamment lorsque la seringue est remplie manuellement juste avant son utilisation, est que le volume de la dose distribuée n'est pas défini de manière précise et reproductible, ce qui peut générer des variations de dosages non souhaitables si le dispositif est réutilisé plusieurs fois avec différentes seringues.

Le document US4962868 décrit un dispositif selon le préambule de la revendication 1.

La présente invention a pour but de fournir un dispositif de distribution de produit fluide qui ne présente pas les inconvénients susmentionnés.

En particulier, la présente invention a pour but de fournir un dispositif de distribution de produit fluide, tel qu'un unidose, qui soit simple et fiable à utiliser, et dans lequel la distribution du produit est indépendante de l'effort d'actionnement de l'utilisateur.

La présente invention a également pour but de fournir un tel dispositif de distribution qui garantisse la distribution de la totalité d'une dose à chaque actionnement.

La présente invention a aussi pour but de fournir un tel dispositif de distribution qui évite les risques de blessures lors de l'actionnement.

La présente invention a également pour but de fournir un tel dispositif qui utilise une seringue en tant que réservoir et qui permet, lors de l'insertion de la seringue dans le dispositif, de définir de manière reproductible la dose de produit fluide qui sera distribuée lors de l'actionnement.

La présente invention a encore pour but de fournir un tel dispositif de distribution qui soit simple et peu coûteux à fabriquer et à assembler.

La présente invention a donc pour objet un dispositif de distribution de produit fluide comportant :
- un corps recevant un réservoir amovible, ledit réservoir comportant un corps cylindrique contenant une dose de produit fluide et un piston relié à une tige de piston, ledit piston étant déplaçable axialement dans ledit corps cylindrique entre une position de repos et une position de distribution,
- un système d'actionnement comportant une tige d'actionnement déplaçable axialement dans ledit corps entre une position de repos et une position actionnée, ladite tige d'actionnement étant sollicitée vers sa position actionnée par un élément élastique, tel qu'un ressort, ladite tige d'actionnement coopérant avec ladite tige de piston dudit réservoir, de sorte que quand ladite tige d'actionnement est déplacée par ledit élément élastique vers sa position actionnée, ledit piston est déplacé vers sa position de distribution, provoquant la distribution du produit fluide à travers ledit orifice de distribution,
dans lequel :
- ledit corps cylindrique dudit réservoir comporte d'un côté axial des moyens de positionnement et de l'autre côté axial un orifice de distribution,
- ladite tige de piston comporte une projection radiale, notamment à son extrémité axiale opposée audit piston,
- ledit corps comporte des premiers moyens de réception recevant lesdits moyens de positionnement dudit réservoir, et
- ladite tige d'actionnement comporte des seconds moyens de réception recevant ladite projection radiale de ladite tige de piston.

Avantageusement, lesdits moyens de positionnement comportent au moins une ailette radiale de positionnement, lesdits premiers moyens de réception étant réalisés sous la forme d'au moins une rainure radiale recevant chacune une ailette radiale de positionnement respective.

Avantageusement, lesdits seconds moyens de réception comportent un logement radial adapté à la forme de ladite projection radiale.

Avantageusement, le dispositif comporte des moyens de blocage, déplaçables entre une position de blocage, dans laquelle ladite la tige d'actionnement est bloquée en position de repos, et une position de non blocage, lesdits moyens de blocage étant déplacés manuellement par l'utilisateur entre ladite position de blocage et ladite position de non blocage.

Avantageusement, lesdits moyens de blocage comportent au moins une patte élastique solidaire de ladite tige d'actionnement, chaque patte élastique étant sollicitée radialement vers l'extérieur et étant élastiquement déformable radialement vers l'intérieur, chaque patte élastique coopère en position de blocage avec un épaulement radial respectif.

Avantageusement, ledit au moins un épaulement radial est formé dans un manchon creux fixé dans ledit corps, ledit manchon creux comportant une paroi radiale comportant un trou traversant central, ladite tige d'actionnement traversant ledit trou traversant, le bord dudit trou traversant de ladite paroi radiale formant ledit épaulement radial, avec en position de blocage chaque patte élastique qui bute contre un épaulement radial respectif pour empêcher tout déplacement axial de ladite tige d'actionnement.

Avantageusement, lesdits moyens de blocage sont débloqués au moyen d'un bouton d'actionnement, déplaçable axialement par l'utilisateur entre une position de repos et une position de déclenchement.

Avantageusement, ledit bouton d'actionnement comporte un manchon de déclenchement adapté à coopérer avec ladite au moins une patte élastique.

Avantageusement, ledit manchon de déclenchement comporte un diamètre interne inférieur au diamètre du trou traversant formé dans ladite paroi radiale dudit manchon creux.

Ces avantages et caractéristiques et d'autres de la présente invention apparaîtront au cours de la description détaillée suivante donnée à titre d'exemple non limitatif, en regard de dessins joints, sur lesquels :
la figure 1 est une vue schématique en perspective découpée d'un dispositif de distribution de produit fluide selon un mode de réalisation avantageux, sans réservoir et en position de repos du système d'actionnement,
la figure 2 est une vue schématique en section transversale du dispositif de la figure 1,
la figure 3 est une vue schématique en section transversale selon un autre angle de coupe du dispositif de la figure 1,
la figure 4 est une vue similaire à celle de la figure 1, après chargement du système d'actionnement et avant insertion du réservoir,
la figure 5 est une vue schématique en section transversale du dispositif de la figure 4,
la figure 6 est une vue schématique en section transversale selon un autre angle de coupe du dispositif de la figure 4,
la figure 7 est une vue similaire à celle de la figure 4, après insertion du réservoir et avant déclenchement du système d'actionnement,
la figure 8 est une vue schématique en section transversale du dispositif de la figure 7,
la figure 9 est une vue schématique en section transversale selon un autre angle de coupe du dispositif de la figure 7,

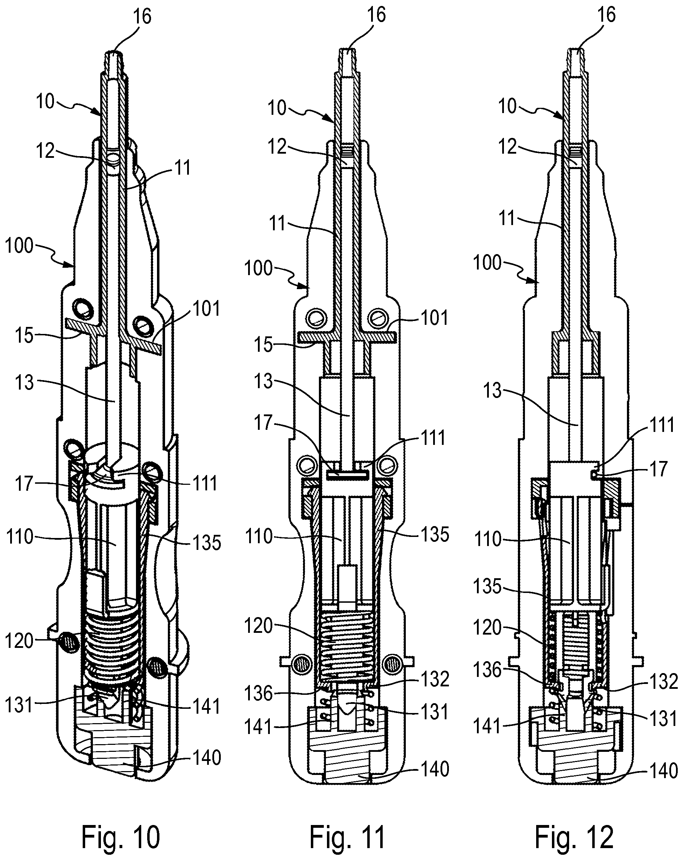

la figure 10 est une vue similaire à celle de la figure 7, après déclenchement du système d'actionnement,
la figure 11 est une vue schématique en section transversale du dispositif de la figure 10,
la figure 12 est une vue schématique en section transversale selon un autre angle de coupe du dispositif de la figure 10,
la figure 13 est une vue similaire à celle de la figure 10, après distribution de la dose de produit fluide,
la figure 14 est une vue schématique en section transversale du dispositif de la figure 13, et
la figure 15 est une vue schématique en section transversale selon un autre angle de coupe du dispositif de la figure 13.

Dans la description ci-après, les termes "axial" et "radial" se réfèrent à l'axe central longitudinal du dispositif.

Le dispositif comporte un corps 100 adapté à recevoir un réservoir 10 contenant une dose de produit fluide, et comportant un système d'actionnement utilisé pour expulser ladite dose de produit fluide hors du réservoir.

Le réservoir 10 comporte un corps cylindrique 11 contenant le produit fluide et un piston 12 relié à une tige de piston 13.

Le piston 12 est déplaçable axialement dans le corps cylindrique 11 entre une position de repos et une position de distribution.

Le corps cylindrique 11 comporte d'un côté axial des moyens de positionnement 15, par exemple au moins une, de préférence deux ailettes radiales de positionnement 15, et de l'autre côté axial un orifice de distribution 16. En variante, les moyens de positionnement 15 pourrait être formés par une collerette radiale.

La tige de piston 13 comporte une projection radiale 17, de préférence à son extrémité axiale opposée au piston 12. Ce réservoir 10 peut être rempli avec le produit à distribuer avant d'être inséré dans le corps 1. En variante, il peut être prérempli.

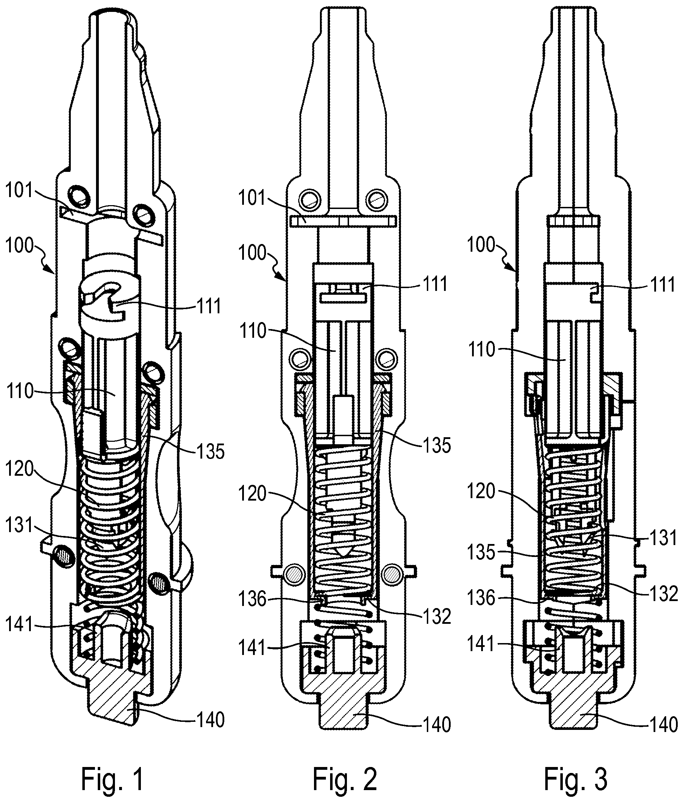

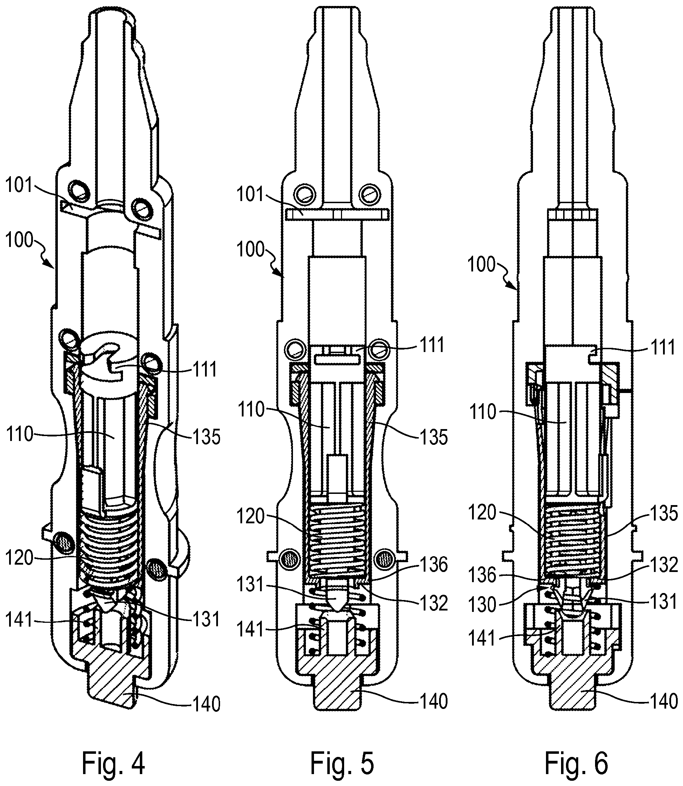

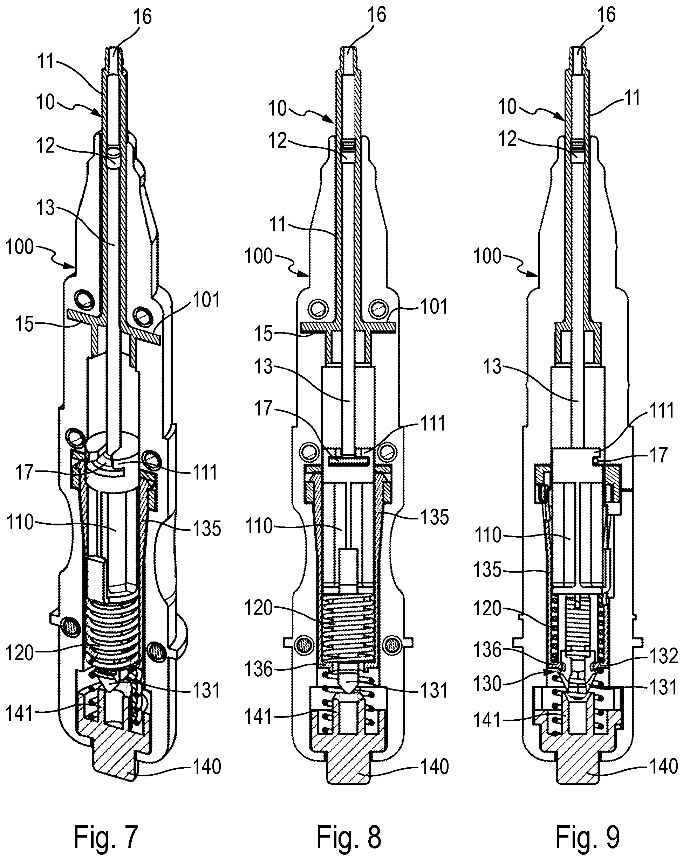

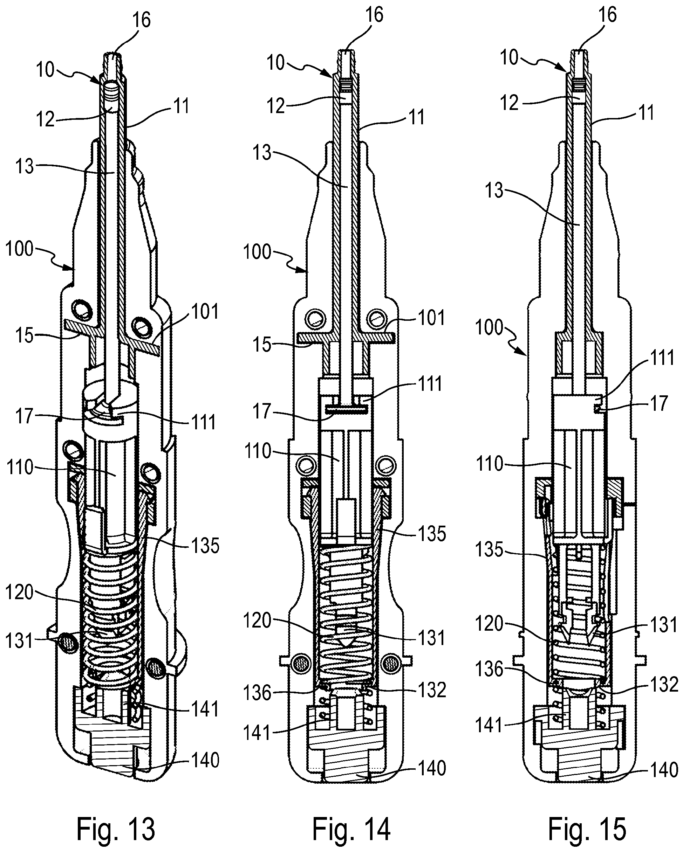

Le système d'actionnement comporte une tige d'actionnement 110 déplaçable axialement dans le corps 100 entre une position de repos, visible sur les figures 1 à 9, et une position actionnée, visible sur les figures 13 à 15.

La tige d'actionnement 110 est sollicitée vers sa position actionnée par un élément élastique 120, tel qu'un ressort.

La tige d'actionnement 110 coopère avec la tige de piston 13 du réservoir 10, de sorte que quand la tige d'actionnement 110 est déplacée par le ressort 120 vers sa position actionnée, le piston 12 est déplacé vers sa position de distribution, provoquant la distribution du produit fluide à travers l'orifice de distribution 16.

De préférence, des moyens de blocage 130, déplaçables entre une position de blocage et une position de non blocage, retiennent la tige d'actionnement 110 en position de repos. Ces moyens de blocage 130 sont déplaçables manuellement par l'utilisateur entre la position de blocage et la position de non blocage.

Dans l'exemple des figures, les moyens de blocage 130 comportent au moins une patte élastique 131 solidaire de la tige d'actionnement 110.

Chaque patte élastique 131 est sollicitée radialement vers l'extérieur et est élastiquement déformable radialement vers l'intérieur.

Chaque patte élastique 131 coopère en position de blocage avec un épaulement radial respectif 132.

Dans l'exemple des figures, il y a deux pattes élastiques 131 diamétralement opposées, et les épaulements radiaux respectifs 132 sont formés dans un manchon creux 135 fixé dans le corps 100. Ce manchon creux 135 comporte une paroi radiale 136 comportant un trou traversant central, ladite tige d'actionnement 110 traversant ledit trou traversant.

Le bord dudit trou traversant de la paroi radiale 136 forme l'épaulement radial 132, avec en position de blocage les pattes élastiques 131 qui butent contre ledit épaulement radial 132 pour empêcher tout déplacement axial de la tige d'actionnement 110.

Lorsque l'utilisateur souhaite débloquer les moyens de blocage 130, il déforme lesdites pattes élastiques 131 radialement vers l'intérieur jusqu'à ce qu'elles ne butent plus sur l'épaulement radial 132.

Le ressort 120 déplace alors la tige d'actionnement vers sa position actionnée, avec les pattes élastiques 131 qui passent à travers la paroi de fond trouée du manchon creux 135.

Avantageusement, le dispositif est rechargeable, le système d'actionnement étant rechargé avant chaque utilisation, en tirant ou poussant axialement sur la tige d'actionnement 110, ce qui charge le ressort 120, jusqu'à ce que les pattes élastiques 131 passent à travers la paroi radiale trouée 136 du manchon creux 135 et viennent s'encliqueter derrière l'épaulement radial 132.

Dans l'exemple représenté sur les figures, les moyens de blocage 130 sont avantageusement débloqués au moyen d'un bouton d'actionnement 140, déplaçable axialement par l'utilisateur entre une position de repos et une position de déclenchement.

Le bouton d'actionnement 140 comporte un manchon de déclenchement 141 adapté à coopérer avec les pattes élastiques 131 des moyens de blocage 130. Ce manchon de déclenchement 141 comporte avantageusement un diamètre interne inférieur au diamètre du trou traversant formé dans la paroi radiale 136 du manchon creux 135.

Ainsi, lorsque l'utilisateur appuie sur le bouton d'actionnement 140, il déplace le manchon de déclenchement 141 axialement dans le corps 100, ce qui déforme les pattes élastiques 131 radialement vers l'intérieur jusqu'à ce qu'elles ne coopèrent plus avec l'épaulement radial 132.

D'autres mises en œuvre des moyens de blocage 130 et du bouton d'actionnement 140 sont possibles. Par exemple, le bouton d'actionnement 140 pourrait être déplaçable non pas axialement dans le corps 100, mais selon une direction latérale.

Eventuellement, une tête de distribution, notamment sous forme de spray, peut être prévue, qui peut être assemblée sur le corps 100 et/ou sur le réservoir 10.

Selon l'invention, le corps 100 comporte des premiers moyens de réception 101 recevant les moyens de positionnement 15 du réservoir 10. Ainsi, le corps cylindrique 11 du réservoir 10 est positionné de manière déterminée et reproductible dans le corps 100 du dispositif. Ces premiers moyens de réception 101 sont par exemple réalisés sous la forme de rainures radiales recevant chacune une ailette de positionnement 15.

Par ailleurs, la tige d'actionnement 110 comporte des seconds moyens de réception 111 recevant la projection radiale 17 de la tige de piston.

Ainsi, la tige de piston 13 et le piston 12 du réservoir 10 sont positionnés de manière déterminée et reproductible dans le corps 100 du dispositif.

Ces seconds moyens de réception 111 sont par exemple réalisés sous la forme d'un logement radial adapté à la forme de la projection radiale 17.

Ainsi, la présente invention définit de manière précise et reproductible la position du piston 12 dans le corps cylindrique 11 du réservoir, et donc la dose de produit fluide qui sera expulsée lors de l'actionnement. Ceci est bénéfique notamment pour garantir des caractéristiques de distribution toujours identiques, puisque le volume du produit fluide distribué est toujours identique et la force d'actionnement par le ressort 120 est toujours identique.

La présente invention fournit donc un dispositif de distribution qui assure notamment les fonctions suivantes :
- il assure une sécurité d'utilisation en permettant la distribution du produit fluide dans n'importe quelle position du dispositif ;
- il facilite la distribution du produit qui est indépendante de l'effort de l'utilisateur, puisque c'est un ressort qui assure cette distribution ; en particulier, le ressort garantit des caractéristiques de distribution constantes ;
- il limite les risques de contamination du produit en mettant celui-ci en contact avec seulement deux matériaux, à savoir le matériau du réservoir, en général du verre, et le matériau du piston, en général un matériau élastomère inerte vis-à-vis du produit fluide.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant :
- un corps (100) recevant un réservoir (10) amovible, ledit réservoir (10) comportant un corps cylindrique (11) contenant une dose de produit fluide et un piston (12) relié à une tige de piston (13), ledit piston (12) étant déplaçable axialement dans ledit corps cylindrique (11) entre une position de repos et une position de distribution,
- un système d'actionnement comportant une tige d'actionnement (110) déplaçable axialement dans ledit corps (100) entre une position de repos et une position actionnée, ladite tige d'actionnement (110) étant sollicitée vers sa position actionnée par un élément élastique (120), tel qu'un ressort, ladite tige d'actionnement (110) coopérant avec ladite tige de piston (13) dudit réservoir (10), de sorte que quand ladite tige d'actionnement (110) est déplacée par ledit élément élastique (120) vers sa position actionnée, ledit piston (12) est déplacé vers sa position de distribution, provoquant la distribution du produit fluide à travers ledit orifice de distribution (16),
**caractérisé en ce que** :
- ledit corps cylindrique (11) dudit réservoir (10) comporte d'un côté axial des moyens de positionnement (15) et de l'autre côté axial un orifice de distribution (16),
- ladite tige de piston (13) comporte une projection radiale (17), notamment à son extrémité axiale opposée audit piston (12),
- ledit corps (100) comporte des premiers moyens de réception (101) recevant lesdits moyens de positionnement (15) dudit réservoir (10), et
- ladite tige d'actionnement (110) comporte des seconds moyens de réception (111) recevant ladite projection radiale (17) de ladite tige de piston (13).

2. Dispositif selon la revendication 1, dans lequel lesdits moyens de positionnement (15) comportent au moins une ailette radiale de positionnement, lesdits premiers moyens de réception (101) étant réalisés sous la forme d'au moins une rainure radiale recevant chacune une ailette radiale de positionnement (15) respective.

3. Dispositif selon la revendication 1 ou 2, dans lequel lesdits seconds moyens de réception (111) comportent un logement radial adapté à la forme de ladite projection radiale (17).

4. Dispositif selon l'une quelconque des revendications précédentes, comportant des moyens de blocage (130), déplaçables entre une position de blocage, dans laquelle ladite la tige d'actionnement (110) est bloquée en position de repos, et une position de non blocage, lesdits moyens de blocage (130) étant déplacés manuellement par l'utilisateur entre ladite position de blocage et ladite position de non blocage.

5. Dispositif selon la revendication 4, dans lequel lesdits moyens de blocage (130) comportent au moins une patte élastique (131) solidaire de ladite tige d'actionnement (110), chaque patte élastique (131) étant sollicitée radialement vers l'extérieur et étant élastiquement déformable radialement vers l'intérieur, chaque patte élastique (131) coopère en position de blocage avec un épaulement radial respectif (132).

6. Dispositif selon la revendication 5, dans lequel ledit au moins un épaulement radial (132) est formé dans un manchon creux (135) fixé dans ledit corps (100), ledit manchon creux (135) comportant une paroi radiale (136) comportant un trou traversant central, ladite tige d'actionnement (110) traversant ledit trou traversant, le bord dudit trou traversant de ladite paroi radiale (136) formant ledit épaulement radial (132), avec en position de blocage chaque patte élastique (131) qui bute contre un épaulement radial (132) respectif pour empêcher tout déplacement axial de ladite tige d'actionnement (110).

7. Dispositif selon l'une quelconque des revendications 4 à 6, dans lequel lesdits moyens de blocage (130) sont débloqués au moyen d'un bouton d'actionnement (140), déplaçable axialement par l'utilisateur entre une position de repos et une position de déclenchement.

8. Dispositif selon la revendication 7, dans lequel ledit bouton d'actionnement (140) comporte un manchon de déclenchement (141) adapté à coopérer avec ladite au moins une patte élastique (131).

9. Dispositif selon la revendication 8, dans lequel ledit manchon de déclenchement (141) comporte un diamètre interne inférieur au diamètre du trou traversant formé dans ladite paroi radiale (136) dudit manchon creux (135).

## Patentansprüche

1. Vorrichtung zur Abgabe von fluidem Produkt, aufweisend:
- einen Körper (100), der einen herausnehmbaren Behälter (10) aufnimmt, wobei der Behälter (10) einen zylindrischen Körper (11) aufweist, der eine Dosis flüssigen Produkts enthält, und einen Kolben (12), der mit einer Kolbenstange (13) verbunden ist, wobei der Kolben (12) axial in dem zylindrischen Körper (11) zwischen einer Ruheposition und einer Abgabeposition beweglich ist,
- ein Betätigungssystem, das eine Betätigungsstange (110) aufweist, die axial in dem Körper (100) zwischen einer Ruheposition und einer betätigten Position beweglich ist, wobei die Betätigungsstange (110) durch ein elastisches Element (120), beispielsweise eine Feder, in ihre betätigte Position beansprucht wird, wobei die Betätigungsstange (110) mit der Kolbenstange (13) des Behälters (10) derart zusammenwirkt, dass, wenn die Betätigungsstange (110) durch das elastische Element (120) in ihre betätigte Position bewegt wird, der Kolben (12) in seine Abgabeposition bewegt wird, wodurch die Abgabe des fluiden Produkts durch die Abgabeöffnung (16) erfolgt,
**dadurch gekennzeichnet, dass**:
- der zylindrische Körper (11) des Behälters (10) auf einer axialen Seite Positionierungsmittel (15) und auf der anderen axialen Seite eine Abgabeöffnung (16) umfasst,
- die Kolbenstange (13) einen radialen Vorsprung (17), insbesondere an ihrem axialen Ende, das dem Kolben (12) gegenüberliegt, aufweist,
- der Körper (100) erste Empfangsmittel (101) aufweist, die die Positionierungsmittel (15) des Behälters (10) aufnehmen, und
- die Betätigungsstange (110) zweite Empfangsmittel (111) aufweist, die den radialen Vorsprung (17) der Kolbenstange (13) aufnehmen.

2. Vorrichtung nach Anspruch 1, wobei die Positionierungsmittel (15) mindestens einen radialen Positionierungsflügel aufweisen, wobei die ersten Empfangsmittel (101) in Form von mindestens einer radialen Nut ausgebildet sind, die jeweils eine entsprechende radiale Positionierungsrippe (15) aufnimmt.

3. Vorrichtung nach Anspruch 1 oder 2, wobei die zweiten Empfangsmittel (111) eine radiale Aufnahme umfassen, die an die Form des radialen Vorsprungs (17) angepasst ist.

4. Vorrichtung nach einem der vorhergehenden Ansprüche, die Arretiermittel (130) aufweist, die zwischen einer Arretierposition, in der die Betätigungsstange (110) in der Ruheposition arretiert ist, und einer Nichtarretierposition beweglich ist, wobei die Arretiermittel (130) von dem Benutzer manuell zwischen der Arretierposition und der Nichtarretierposition bewegt werden.

5. Vorrichtung nach Anspruch 4, wobei die Arretiermittel (130) mindestens eine elastische Lasche (131) aufweisen, die mit der Betätigungsstange (110) fest verbunden ist, wobei jede elastische Lasche (131) radial nach außen beansprucht und radial nach innen elastisch verformbar ist und jede elastische Lasche (131) in der Arretierposition mit einer entsprechenden radialen Schulter (132) zusammenwirkt.

6. Vorrichtung nach Anspruch 5, wobei die mindestens eine radiale Schulter (132) in einer Hohlhülse (135) ausgebildet ist, die in dem Körper (100) befestigt ist, wobei die Hohlhülse (135) eine radiale Wand (136) aufweist, die eine zentrale Durchgangsbohrung aufweist, wobei die Betätigungsstange (110) die Durchgangsbohrung durchquert, wobei die Kante der Durchgangsbohrung der radialen Wand (136) die radiale Schulter (132) bildet, wobei in der Arretierposition jede elastische Lasche (131) an einer jeweiligen radialen Schulter (132) anliegt, um jedwede axiale Bewegung der Betätigungsstange (110) zu verhindern.

7. Vorrichtung nach einem der Ansprüche 4 bis 6, wobei die Arretiermittel (130) mittels eines Betätigungsknopfes (140) freigegeben werden, der von dem Benutzer axial zwischen einer Ruheposition und einer Auslöseposition bewegbar ist.

8. Vorrichtung nach Anspruch 7, wobei der Betätigungsknopf (140) eine Auslösehülse (141) aufweist, die zum Zusammenwirken mit der mindestens einen elastischen Lasche (131) ausgebildet ist.

9. Vorrichtung nach Anspruch 8, wobei die Auslösehülse (141) einen Innendurchmesser aufweist, der kleiner als der Durchmesser der in der radialen Wand (136) der Hohlhülse (135) ausgebildeten Durchgangsbohrung ist.

## Claims

1. A device for dispensing a fluid product comprising:
- A body (100) receiving a removable reservoir (10), said reservoir (10) comprising a cylindrical body (11) containing a dose of fluid product and a plunger (12) connected to a plunger rod (13), said plunger (12) being axially movable in said cylindrical body (11) between a rest position and a dispensing position,
- an actuation system comprising an actuation rod (110) axially movable in said body (100) between a rest position and an actuated position, said actuation rod (110) being urged towards its actuated position by an elastic element (120), such as a spring, said actuation rod (110) cooperating with said plunger rod (13) of said reservoir (10), so that when said actuation rod (110) is moved by said elastic element (120) towards its actuated position, said plunger (12) is moved towards its dispensing position, causing the dispensing of the fluid product through said dispensing orifice (16),
**characterised in that**:
- said cylindrical body (11) of said reservoir (10) comprises, on one axial side, positioning means (15) and on the other axial side, a dispensing orifice (16),
- said plunger rod (13) comprises a radial projection (17), particularly, at its axial end opposite said plunger (12),
- said body (100) comprises first receiving means (101), receiving said positioning means (15) of said reservoir (10), and
- said actuation rod (110) comprises second receiving means (111), receiving said radial projection (17) of said plunger rod (13).

2. The device according to claim 1, wherein said positioning means (15) comprise at least one radial positioning fin, said first receiving means (101) being made in the form of at least one radial groove each receiving a respective radial positioning fin (15).

3. The device according to claim 1 or claim 2, wherein said second receiving means (111) comprise a radial housing adapted to the shape of said radial projection (17).

4. The device according to any one of the preceding claims, comprising blocking means (130) movable between a blocking position, wherein said actuation rod (110) is blocked in the rest position, and a non-blocking position, said blocking means (130) being moved manually by the user between said blocking position and said non-blocking position.

5. The device according to claim 4, wherein said blocking means (130) comprise at least one elastic tab (131) integral with said actuation rod (110), each elastic tab (131) being urged radially outwards and being elastically deformable radially inwards, each elastic tab (131) cooperates in the blocking position with a respective radial shoulder (132).

6. The device according to claim 5, wherein said at least one radial shoulder (132) is formed in a hollow sleeve (135) fixed in said body (100), said hollow sleeve (135) comprising a radial wall (136) comprising a central through-hole, said actuation rod (110) passing through said through-hole, the edge of said through-hole of said radial wall (136) forming said radial shoulder (132), with, in the blocking position, each elastic tab (131) which abuts against a respective radial shoulder (132) to prevent said actuation rod (110) all axial movement of said actuation rod (110)

7. The device as claimed in any one of claims 4 to 6, wherein said blocking means (130) are unblocked by means of an actuation button (140) axially movable by the user between a rest position and a trigger position.

8. The device according to claim 7, wherein said actuation button (140) comprises a trigger sleeve (141) adapted to cooperate with said at least one elastic tab (131).

9. The device according to claim 8, wherein said trigger sleeve (141) has an internal diameter smaller than the diameter of the through-hole formed in said radial wall (136) of said hollow sleeve (135).
